(19) Europäisches Patentamt
European Patent Office
Office européen des brevets

(11) Veröffentlichungsnummer: **0 505 597 A1**

## (12) EUROPÄISCHE PATENTANMELDUNG

(21) Anmeldenummer: **91104978.1**

(51) Int. Cl.5: **A61B 5/087**

(22) Anmeldetag: **28.03.91**

(43) Veröffentlichungstag der Anmeldung:
**30.09.92 Patentblatt 92/40**

(84) Benannte Vertragsstaaten:
**AT BE CH DE DK ES FR GB GR IT LI LU NL SE**

(71) Anmelder: **Pohl, Josef Peter, Dipl.-Ing. (FH)**
**38, Kreuzschanze**
**W-6500 Mainz 1(DE)**

(72) Erfinder: **Pohl, Josef Peter, Dipl.-Ing. (FH)**
**38, Kreuzschanze**
**W-6500 Mainz 1(DE)**

(74) Vertreter: **Kodron, Rudolf S., Dipl.-Ing.**
**Adam-Karrillon-Strasse 30**
**W-6500 Mainz(DE)**

(54) **Instrument zur Messung der Atmungskapazität (Pneumotachometer).**

(57) Ein Instrument zur Messung der Atmungskapazität (Pneumotachometer), bestehend aus einem Gehäuse (1, 12) mit zwei Öffnungen (14, 11) für den Einlaß und den Auslaß der Ausatemluft, sowie einem im Gehäuse drehbar gelagerten, federbelasteten, durch die Atmungsluft bewegbaren Schwenkarm (3), dessen Schwenkwinkel durch eine angeschlossene Zeiger-Skala-Meßeinrichtung (9, 10) angezeigt wird, soll dahingehend verbessert werden, daß kein anfänglicher Druckaufbau nötig ist und daß die fortschreitende Vergrößerung der Durchlaßweite für die Ausatemluft nicht am Auslaßende des Instruments, sondern in dessen Innenbereich stattfindet, so daß bei einer konstant bleibenden Größe der Auslaßöffnung von Anfang an der Aufbau einer Luftströmungsbewegung im Instrument möglich ist.

Erreicht wird dies dadurch, daß das Gehäuse - abgesehen von einem eingesetzten trichterförmigen Mundstück- quaderförmig ausgebildet ist, die Öffnungen für den Einlaß und den Auslaß der Ausatemluft in zwei gegenüberliegenden Gehäusewandungen angeordnet sind und der in den Luftdurchströmungskanal hineinragende Schwenkarm so groß bemessen ist, daß er in seiner Ausgangsstellung (Nullpunktlage) die freie Querschnittsfläche des Luftdurchströmungskanals nicht vollständig verschließt.

EP 0 505 597 A1

# Fig.1

Die Erfindung betrifft ein Instrument zur Messung der Atmungskapazität (Pneumotachometer), bestehend aus einem Gehäuse mit zwei Öffnungen für den Einlaß und den Auslaß der Ausatemluft, sowie einem im Gehäuse drehbar gelagerten, federbelasteten, durch die Atmungsluft bewegbaren Schwenkarm, dessen Ausschwenkwinkel durch eine angeschlossene Zeiger-Skala-Meßeinrichtung angezeigt wird.

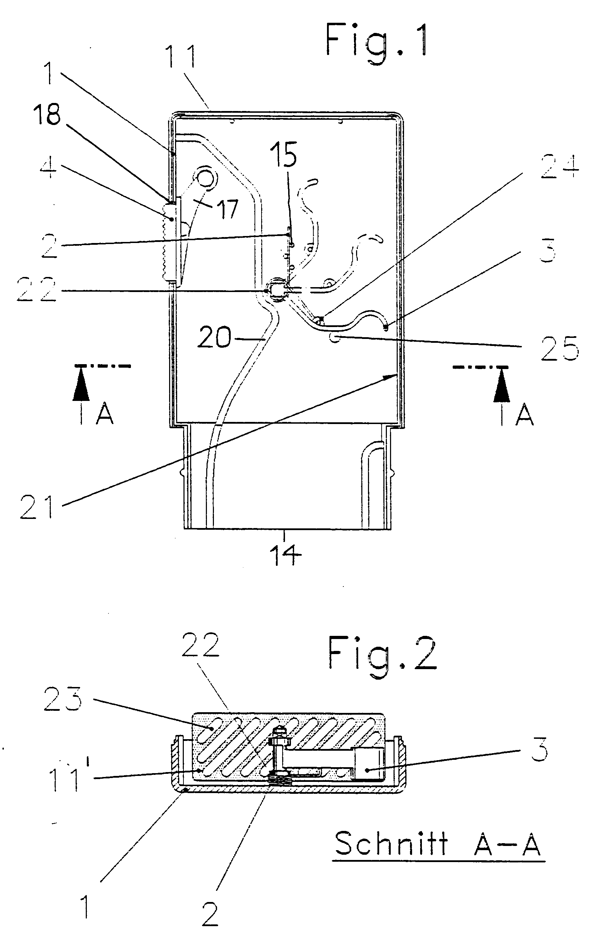

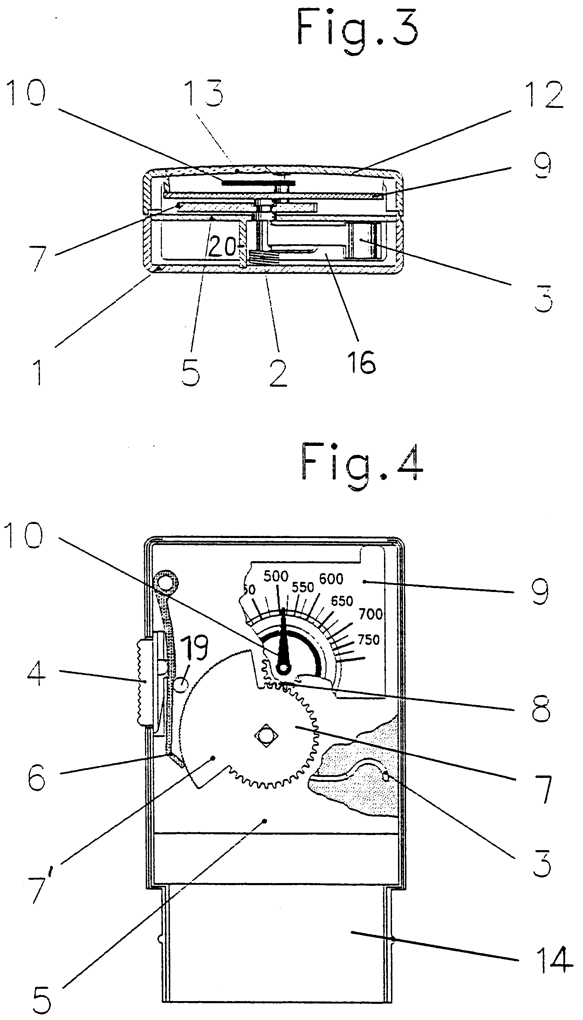

Ein Instrument der genannten Art wird beispielsweise in den Figuren 2 und 3 im deutschen Gebrauchsmuster 72 16 404 beschrieben. Dieses dosenförmige Instrument hat den Nachteil, daß der Schwenkarm in der Nullpunktlage den Luftdurchströmungskanal vollkommen verschließt, so daß sich erst ein Druck aufbauen muß, welcher den Beginn der Schwenkbewegung des Schwenkarms verursacht, der anschließend in seiner Schwenkbewegung die Auslaßöffnung fortschreitend vergrößert.

Der Erfindung liegt die Aufgabe zugrunde, dieses Instrument in der Richtung zu verbessern, daß kein anfänglicher Druckaufbau nötig ist und daß die fortschreitende Vergrößerung der Durchlaßweite für die Ausatemluft nicht am Auslaßende des Instruments, sondern in dessen Innenbereich stattfindet, so daß bei einer konstant bleibenden Größe der Auslaßöffnung von Anfang an der Aufbau einer Luftströmungsbewegung im Instrument möglich ist.

Gelöst wird diese Aufgabe durch die im kennzeichnenden Teil des Patentanspruchs 1 angegebene Konstruktion.
Vorteilhafte Ausgestaltungen der Erfindung sind Gegenstand der Unteransprüche.

Das erfindungsgemäße Instrument zeichnet sich durch kompakte Konstruktion -etwa in Größe und Form einer Zigarettenschachtel- sowie einfache und wirtschaftliche Herstellbarkeit aus. Die Kompaktheit des Instruments ergibt sich aus der quaderförmigen Gehäuseform und der Tatsache, daß alle beweglichen Teile innerhalb des Gehäuses untergebracht sind. Dadurch ist das Instrument auch handhabungssicher.

Ein Ausführungsbeispiel der Erfindung wird nachstehend mit Bezug auf die Zeichnungen mehr im einzelnen beschrieben.

Die Darstellung gemäß Figur 1 zeigt eine Draufsicht auf das Instrument, wobei lediglich die Gehäuseunterhälfte und die von dieser fixierten Instrumententeile gezeigt und die übrigen Teile fortgelassen sind.

Die Figur 2 zeigt einen Querschnitt entlang Schnittlinie A-A des Gegenstandes nach Figur 1.

Die Figur 3 zeigt eine Querschnittsdarstellung durch das gesamte fertig zusammengebaute Instrument ohne Darstellung der Arretierungseinrichtung für den Zeiger.

Die Figur 4 zeigt eine Draufsicht auf das Instrument ohne die obere Gehäusehälfte und mit teilweise weggebrochenen Gehäusezwischenwänden 5 und 9.

Die Darstellung gemäß Figur 1 zeigt die quaderförmige Gehäuseunterhälfte 1 und die in gegenüberliegenden Gehäusewandungen angeordnete Einlaßöffnung 14 und Auslaßöffnung 11 der Ausatemluft.

Etwa in der Mitte der Gehäuseunterhälfte 1 trägt diese einen hohlzylindrischen Ansatz 22, um den sich außen die Feder 2 windet, deren eines Ende durch Arretierungsnocken 15 der Gehäuseunterhälfte 1 fixiert ist, wogegen das andere Ende der Feder vertikal umgebogen ist und durch das Einsteck-Augloch 24 des Schwenkarms gehalten wird, der wiederum mit seiner Drehachse im freien Innenraum des hohlzylindrischen Ansatz 22 gelagert ist.

Es ist klar, daß mit zunehmender Schwenkbewegung des Schwenkarms 3 der Abstand zwischen dessen freiem Ende und der ebenen Gehäuseinnenwandfläche 21 sich ständig vergrößert, bis die Schwenkbewegung infolge Ausgleichs zwischen der hindurchge - blasenen und der durch den Luftdruck durch die Auslaßöffnung 11 hereingedrückten Luft zum Stillstand kommt. Diese maximale Ausschwenkbewegung wird durch eine Zeiger-Skala-Meßeinrichtung angezeigt und festgehalten, die später erläutert wird.

Die Querschnittsdarstellung gemäß Figur 2 zeigt, daß die Auslaßöffnung aus einer Mehrzahl von Einzeldurchbrechungen 23 in einer Abschlußwand 11' gebildet wird.

Der Darstellung gemäß Figur 3 ist zu entnehmen, daß der Schwenkarm 3 derart ausgebildet ist, daß er den durch die Trennwand 20 und die Gehäusezwischenwand 5 begrenzten Luftdurchströmungskanal 16 nicht vollständig verschließt, so daß von Anfang an die eingeblasene Ausatemluft strömen kann.

Am oberen Ende der Schwenkachse des Schwenkarms 3 ist ein großes Zahnrad 7 befestigt, in welches die Zähne eines weiteren in Figur 4 dargestellten kleinen Zahnrads 8 eingreifen. Das kleine Zahnrad 8 trägt oberseitig und oberhalb einer waagrechten Skalenzwischenwand 9 einen Meßzeiger 10. Es ist klar, daß über eine geringe Schwenkbewegung des Schwenkarms 3 das große Zahnrad 7 bewegt wird und daß diese Schwenkbewegung infolge der Übersetzung auf das kleine Zahnrad 8 zu einer starken Verschwenkung des Zeigers 10 führt.

Da die Gehäuseoberhälfte 12 einen transparenten Abschnitt 13 aufweist, wird hierdurch die Stellung des Zeigers 10 auf der Skalenzwischenwand 9 sichtbar.

Da das große Zahnrad 7 einen Sektor 7' mit vergrößertem Radius besitzt, in welchen das freie Ende einer in der Trennwand 5 schwenkbar gelagerten Klinke 6 eingreift, bleibt der Zeiger 10 in seiner maximalen Stellung stehen.

Die besagte Klinke 6 wird durch Fingerdruck auf den Druckknopf 4 eines Schwenkhebels 17, der in der Gehäuseunterhälfte 1 schwenkbar gelagert ist, durchgebogen, da die Klinke 6 gegen einen als Anschlag dienenden Vorsprung 19 der Gehäusezwischenwand 5 anstößt, so daß das freie Ende der Klinke 6 durch diese Klinkenverbiegung außer Eingriff im Sektor 7' gerät. Die Feder 2 bewegt danach den Schwenkarm 3 und den auf diese Weise freigegebenen Zeiger 10 wieder in die Ausgangsstellung (Null-Punktlage) zurück.

**Patentansprüche**

1. Instrument zur Messung der Atmungskapazität (Pneumotachometer), bestehend aus einem Gehäuse mit zwei Öffnungen für den Einlaß und den Auslaß der Ausatemluft sowie einem im Gehäuse drehbar gelagerten, federbelasteten, durch die Atmungsluft bewegbaren Schwenkarm, dessen Ausschwenkwinkel durch eine angeschlossene Zeiger-Skala-Meßeinrichtung angezeigt wird
   dadurch gekennzeichnet, daß
   - das Gehäuse (1,12,13) -abgesehen von einem eingesetzten trichterförmigen Mundstück- quaderförmig ausgebildet ist,
   - die Öffnungen für den Einlaß (14) und den Auslaß (11) der Ausatemluft in zwei gegenüberliegenden Gehäusewandungen angeordnet sind

     und
   - der in den Luftdurchströmungskanal (15) hineinragende Schwenkarm (3) so groß bemessen ist, daß er in seiner Ausgangsstellung (Nullpunktlage) die freie Querschnittsfläche des Luftdurchströmungskanals (15) nicht vollständig verschließt.

2. Instrument nach Anspruch 1,
   dadurch gekennzeichnet, daß
   - die Zeiger-Skala-Meßeinrichtung (10,9) innerhalb des Gehäuses (1,12,13) untergebracht

     und
   - durch ein transparentes Wandungsteil (13 des Geshäuses beobachtbar ist.

3. Instrument nach Anspruch 1 oder Anspruch 2,
   dadurch gekennzeichnet, daß
   - der Zeiger (10) mit seinem Schwenkbereich unterhalb des transparenten Wandungsteils (13) des Gehäuses sowie oberhalb einer mit einer Skala versehenen Gehäusezwischenwand (9) angeordnet ist
     und
   - am anderen Ende seiner die Gehäusezwischenwand (9) durchgreifenden Achse ein kleines Zahnrad (8) trägt,
   - das mit seinen Zähnen in ein großes im Gehäuse (1,12,13) drehbar gelagertes Zahnrad (7) eingreift.

4. Instrument nach einem oder mehreren der vorausgehenden Ansprüche 1 bis 3,
   dadurch gekennzeichnet, daß
   - im Gehäuse (1,12,13) ein Schwenkhebel (17) drehbar gelagert ist, dessen freies Ende als Druckknopf (4) ausgebildet ist und eine Gehäusewandausnehmung (18) durchgreift

     und
   - der Druckknopf (4) beim Eindrücken eine im Gehäuse angelenkte Klinke (6) gegen einen als Anschlag dienenden Vorsprung (19) einer Gehäusewandung - insbesondere einer Gehäusezwischenwandung (5)- derart andrückt und durchbiegt,
   - daß ihr im druckunbelasteten Zustand im großen Zahnrad (7) eingerastetes freies Ende ausrastet.

5. Instrument nach Anspruch 3 oder 4,
   dadurch gekennzeichnet, daß das große Zahnrad (7) einen Sektor (7') mit vergrößertem Radius und mit kleineren Zähnen für das einrastende freie Ende der Klinke (6) aufweist.

Fig.1

Fig.2

Schnitt A—A

# Fig.3

# Fig.4

Europäisches
Patentamt

**EUROPÄISCHER RECHERCHENBERICHT**

Nummer der Anmeldung

EP    91 10 4978

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | DE-U-9 003 766 (J.P. POHL) <br> * Seite 6, Zeile 1 - Zeile 23; Ansprüche 1-8 * <br> * Abbildungen 1-5 * <br> --- | 1-5 | A61B5/087 |
| A | FR-A-1 234 686 (ETABLISSEMENTS C.A.R.) <br> * Seite 1, linke Spalte, Zeile 14 - Seite 2, linke Spalte, Zeile 6; Abbildungen 1-3 * <br><br> ----- | 1,2 | |
| | | | **RECHERCHIERTE SACHGEBIETE (Int. Cl.5 )** |
| | | | A61B |

Der vorliegende Recherchenbericht wurde für alle Patentansprüche erstellt

| Recherchenort | Abschlußdatum der Recherche | Prüfer |
|---|---|---|
| DEN HAAG | 11 NOVEMBER 1991 | RIEB K.D. |